# EUROPÄISCHE PATENTSCHRIFT

(11) **EP 3 122 290 B1**
(45) Veröffentlichungstag und Bekanntmachung des Hinweises auf die Patenterteilung: **28.08.2019**
(21) Anmeldenummer: 15711796.1
(22) Anmeldetag: 25.03.2015
(51) Int. Cl.: A61B 17/80, A61B 17/56, A61F 2/28, A61F 2/30

(54) **AUGENHÖHLENABDECKGITTER MIT AUSSENKONTURFOLGENDEN LÄNGLICHEN AUSNEHMUNGEN**
EYE SOCKET COVERING GRID WITH LONGITUDINAL RECESSES FOLLOWING EXTERNAL CONTOURS
GRILLE DE RECOUVREMENT DES ORBITES COMPRENANT DES ÉVIDEMENTS LONGITUDINAUX SUIVANT LES CONTOURS EXTÉRIEURS

(30) Priorität: 25.03.2014 EP 14161594
(43) Veröffentlichungstag der Anmeldung: 01.02.2017
(73) Patentinhaber: Karl Leibinger Medizintechnik GmbH & Co. KG, 78570 Mühlheim/Donau (DE)
(72) Erfinder: REINAUER, Frank, 78570 Muehlheim (DE); GRIGORE, Anita, 78570 Muehlheim (DE)
(74) Vertreter: Winter, Brandl, Fürniss, Hübner, Röss, Kaiser, Polte - Partnerschaft mbB
(86) Internationale Anmeldenummer: PCT/EP2015/056426
(87) Internationale Veröffentlichungsnummer: WO 2015/144772

(56) Entgegenhaltungen:
- EP-A1- 2 030 596
- US-A- 5 578 036
- US-A- 5 743 913
- US-A1- 2007 238 069
- US-A1- 2013 172 941

## Beschreibung

### Gebiet der Erfindung

Die Erfindung betrifft ein Augenhöhlenabdeckgitter gemäß des Oberbegriffs des Anspruchs 1, nämlich ein "orbita mesh", mit einem stegausbildenden perforierten Hauptkörper, der eine äußere, normalerweise umlaufende Abschlusskante/Einfassung aufweist.

Das Gitter wird als Anordnung aus länglichen Teilen in regelmäßigen oder unregelmäßigen Abständen verstanden. Es hat eine netzartig ausgestaltete Flächenstruktur.

Aus dem Stand der Technik sind bereits Augenhöhlenabdeckgitter bekannt, wie beispielsweise aus der EP 1 965 735 B1. Dort wird ein Implantat zur Verwendung als Ersatz eines Orbitabodens eingesetzt. Das Implantat ist als Augenhöhlenabdeckgitter ausgestaltet, liegt also am Orbitaboden auf. So ein Implantat, wie ein "Mesh" bzw. Gitter, terkann auch zur seitlichen Orbitarekonstruktion verwendet werden. Es kann auch freitragend eingesetzt werden und muss nicht unbedingt am Boden aufliegen. In der besagten Druckschrift wird ein Implantat für die Verwendung als Ersatz eines Augenhöhlengrunds und optional auch einer medialen Augenhöhlenwandung in der Form einer einstückig vorgeformten Platte, die einen ersten Abschnitt, einen zweiten Abschnitt und einen dritten Abschnitt umfasst, vorgestellt, wobei der erste Abschnitt gemäß einem Augenhöhlengrund und der zweite Abschnitt gemäß einer medialen Seitenwandung geformt sind und der erste Abschnitt und der zweite Abschnitt an einer ersten vorbestimmten Linie anliegen, wobei der dritte Abschnitt zur Befestigung des Implantats am vorderen Augenhöhlenrand angeordnet ist, wobei als besonders herausgestellt ist, dass die erste vorbestimmte Linie eine in der besagten Druckschrift als Bruchlinie definiert ist, entlang welcher ein Arzt ein Segment leicht entfernen kann.

Gitterartig ausgebildete Platten sind auch in ähnlicher Form zum Einsatz an anderen Teilen des Körpers bekannt.

So offenbart beispielsweise die DE 197 46 396 A1 ein Gitter für die Fixierung von Knochenteilen oder für die Überbrückung von Knochenfehlstellen. Ein solches Gitter kann auch am Schädel eingesetzt werden. Letztlich wird in dieser deutschen Druckschrift ein Gitter zur Anwendung im Schädel- und Kieferbereich vorgeschlagen, bestehend aus biokompatiblen Materialien mit einer netzartigen Struktur und mit Ausnehmungen zur Aufnahme von Knochenschrauben, mit denen das Gitter am Knochen befestigbar ist.

Die Stege bilden mäanderförmige, durchgehende, periodische Stegreihen entlang der Hauptachse des Gitters.

Weiterer Stand der Technik ist aus der US 2013/172941 A1, der EP 2 030 596 A1, der US 5,743,913 A, der US 5,578,036 A, und der US 2007/0238069 A1 bekannt. Die EP 2 030 596 A1 offenbart ein Implantat zur Behandlung von Knochen, insbesondere zur Abdeckung von Defekten oder Bohrlöchern oder zur Rekonstruktion von Knochendefekten und Fehlbildungen, insbesondere von Frakturen eines Orbitabodens. Das Implantat umfasst eine Stützstruktur und mindestens einen insbesondere flächigen Anpassungsbereich, wobei die Stützstruktur einen Innenbereich aufweist, der innerhalb eines Randes des Implantats angeordnet ist.

Das Augenhöhlenabdeckgitter, also jene Vorrichtung, die zum In-Kontakt-Gelangen mit dem Orbitaboden vorgesehen ist, darf bei Anbringung am Knochen die Augapfelaufnahme nicht behindern. Jene Augapfelaufnahme ist jedoch nicht sphärisch, sondern erstreckt sich länglich, insbesondere s-förmig.

Die aus dem Stand der Technik bekannten Augenhöhlenabdeckgitter sind leider häufig zu groß, nicht angepasst an den jeweils zu behandelnden individuellen Schädelknochen und auch häufig schwierig anzupassen.

Es ist die Aufgabe der Erfindung, hier Abhilfe zu bieten und eine gute Ausgangsstruktur eines Augenhöhlenabdeckgitters dem Operateur zur Verfügung zu stellen, insbesondere ein solches Augenhöhlenabdeckgitter, was nicht zu groß ist, bereits vorangepasst an den jeweiligen zu behandelnden Schädel ist und einfach feinanpassbar ist. Ferner soll ein Verfahren vorgestellt werden, das ein einfaches Herstellen eines solchen Augenhöhlenabdeckgitters ermöglicht. Letztlich soll auch ein Verfahren vorgestellt werden, um schnell und präzise Verletzungen des Orbitabodens dauerhaft zu behandeln.

### Offenbarung der Erfindung

Diese Aufgabe wird erfindungsgemäß durch ein Augenhöhlenabdeckgitter der gattungsgemäßen Art dadurch gelöst, dass erfindungsgemäß die Stege und Ausnehmungen zur Ausbildung einer imaginären Trennlinie ausgerichtet und beschaffen sind, um eine Sollbruchlinie vorzugeben.

Die Stege und Ausnehmungen sind so angeordnet/ausgerichtet und beschaffen, dass die Außenmaße des Hauptkörpers auf eine individuelle Augenhöhle, vorzugsweise manuell, werkzeuglos oder unter Einsatz einer Zange, anpassbar sind.

Die Anpassbarkeit wird erleichtert, wenn die Stege und Ausnehmungen zur Ausbildung einer imaginären Trennlinie ausgerichtet und beschaffen sind, um eine Sollbruchlinie vorzugeben.

Durch Wegschneiden von Stegen lässt sich die Größe des Orbitabodengitters/Augenhöhlenabdeckgitters einfach an die zu versorgende Stelle anpassen. Der Einsatz einer Konturhilfe zum Ankonturieren ist möglich und wünschenswert.

Unter einem "Folgen der Abschlusskante" wird hier ein paralleles Folgen verstanden, also das pro Abschnitt "in derselben Richtung ausgerichtet sein".

Vorteilhafte Ausführungsformen sind in den Unteransprüchen beansprucht und werden nachfolgend näher erläutert.

Gemäß der Erfindung wird ein Augenhöhlenabdeckgitter dadurch gebildet, dass in Richtung des Zentrums des Gitters eine oder mehrere, längliche Ausnehmungen/Leerräume vorhanden sind, die die gleiche Ausrichtung wie die jeweils benachbarte/nächstgelegene erste Ausnehmung aufweist/aufweisen. Die gesamte, das Gitter ausbildende Platte, ergo der Hauptkörper, ist perforiert. Der Einsatz einer Titanlegierung als Material ist dabei von besonderem Vorteil in puncto Stabilität und Biokompatibilität.

Es ist auch von Vorteil, wenn die innersten, zweiten Ausnehmungen einer Bügeleisensohlenaußenkontur folgend angeordnet sind oder dreieckig ausgerichtet sind. Die innersten zweiten Ausnehmungen sind also besipielsweise bügeleisensohlenaußenkonturartig angeordnet. Sie laufen von einer Grundfläche gesehen geschwungen / konvex in Richtung einer Spitze zu.

Es ist zweckmäßig, wenn der Hauptkörper einen Erweiterungsbereich aufweist, in dem zwei erste Ausnehmungen in einem spitzen Winkel zueinander, etwa 40° bis etwa 70°, vorzugsweise ca. 45°, ca. 50° oder ca. 55° aufeinander zulaufend, angeordnet/ausgerichtet sind.

Ein vorteilhaftes Ausführungsbeispiel ist auch dadurch gekennzeichnet, dass wenigstens eine beispielsweise radial vom Hauptkörper abstehende und/oder am Außenumfang vorhandene/abstehende Lasche, vorzugsweise mehrere Laschen, vorhanden ist/vorhanden sind, die zur Aufnahme von Befestigungsmitteln, wie einer oder mehreren Schrauben, mit einem oder mehreren vorzugsweise kreisrund ausgestalteten Löchern versehen ist/versehen sind. Unter Einsatz bewährter Befestigungsmittel lässt sich dann während der Operation einfach die Fixierung des Augenhöhlenabdeckgitters am Schädelknochen realisieren.

Wenn die im Hauptkörper vorhandenen ersten und zweiten Ausnehmungen überwiegen, also etwa mehr als 50% oder besser mehr als 80%, vorzugsweise 85% bis 95% aller im Hauptkörper enthaltenen Ausnehmungen, eine längliche Kontur aufweisen, so wird eine besonders gute Anpassbarkeit erreicht.

Dabei ist es von Vorteil, wenn ein an eine radial abstehende Lasche angrenzendes Rundloch zwischen zwei ersten Ausnehmungen vorhanden ist. Eine zusätzliche Befestigung im Bereich des Hauptkörpers zur Anbringung am Knochen lässt sich dann realisieren. Die Belastbarkeit des Augenhöhlenabdeckgitters erhöht sich dadurch.

Auch ist es von Vorteil, wenn die Länge der ersten oder zweiten Ausnehmung mehr als 2,5-mal so groß wie die möglichst gleichbleibende Breite ist, vorzugsweise mehr als 3-mal, 3,5-mal oder 4-mal so groß, aber kleiner als das Zehnfache der Breite. Die Belastbarkeit des Augenhöhlenabdeckgitters steht dann in einem guten Verhältnis zur Fluiddurchlässigkeit.

Wenn das Gitter eine mehrfach gekrümmte Raumform einnimmt, vorzugsweise in allen drei Raumrichtungen gekrümmt ist, beispielsweise einer S-Form (im Querschnitt) folgt, so kann ein abstandsloses Anbringen des Augenhöhlenabdeckgitters am Orbitaboden erreicht werden.

Damit die Spannungslinien gut verlaufen, ist es von Vorteil, wenn sich eine zweite Ausnehmung bis zu einem die Abschlusskante/Einfassung ausformenden Steg erstreckt.

Wenn innerhalb der ersten und (auch) zweiten Ausnehmungen ein perforationsloser Mittelsteg vorhanden ist, der etwa im Zentrum des Gitters bzw. des Hauptkörpers angeordnet ist, so kann die Position des Auges besonders gut stabilisiert werden, insbesondere wenn dieser flächig wirkende Mittelsteg in Richtung Posterior ausgerichtet ist/eingesetzt ist.

Damit das Augenhöhlenabdeckgitter nicht zu dick aufträgt, ist es von Vorteil, wenn zumindest der Hauptkörper, vorzugsweise auch die Lasche(n) eine gleichbleibende Dicke, etwa von ca. 0,2 mm bis 0,5 mm, vorzugsweise ca. 0,4 mm aufweist/aufweisen. Der Hauptkörper und die Lasche können insbesondere als integrale Bestandteile einstückig ausgebildet sein. Der Hauptkörper und die Lasche(n) sind also als Monolithteil ausgebildet.

Die Erfindung betrifft letztlich auch ein Verfahren zum Herstellen eines Augenhöhlenabdeckgitters etwa der erfindungsgemäßen Art, wobei unter Nutzung von individuellen Patientendaten eine Negativform / Konturhilfe / patientenspezifisch ausgeformte Schablone geschaffen wird, wobei dann ein vorgefertigtes, beispielsweise platten- und/oder gitterartiges, flächiges und bedarfsweise ebenes Rohstück/Ausgangsbauteil/Urfomteil mit ersten und zweiten länglichen Ausnehmungen auf die Negativform aufgelegt wird, dann unter Aufbringung von Kraft/Druck und/oder Wärme das Rohstück an die Negativform angepasst wird und das abgeformte/fertige Augenhöhlenabdeckgitter von der Negativform abgenommen/vereinzelt wird.

Auch ist die Erfindung einsetzbar bei der medizinischen Behandlung einer beschädigten und/oder deformierten Augenhöhle (Orbita), im Schädel (Cranium) eines Säugetiers, wie eines Menschen, wobei unter Nutzung von CT- oder Röntgentechniken ein Modell des Schädels des Säugetiers, aber auch jedes anderen Tieres, errechnet wird, mit Hilfe dessen und unter Nutzung des erfindungsgemäßen Herstellverfahrens zur Herstellung des erfindungsgemäßen Augenhöhlenabdeckgitters, eine individuell auf das zu behandelnde Säugetier angepasstes fertiges Augenhöhlenabdeckgitter geschaffen wird, das dann am Schädelknochen des zu behandelnden Säugetieres befestigt wird.

Die Erfindung wird nachfolgend mit Hilfe einer Zeichnung näher erläutert. Dabei ist ein erstes Ausführungsbeispiel wiedergegeben. Es zeigen:
- Fig. 1: eine Vorderansicht auf ein erfindungsgemäßes Augenhöhlenabdeckgitter/"orbita mesh",
- Fig. 2: eine Seitenansicht auf das Augenhöhlenabdeckgitter aus Fig. 1,
- Fig. 3: eine Seitenansicht von rechts auf das Augenhöhlenabdeckgitter aus Fig. 1,
- Fig. 4: eine Unteransicht des Augenhöhlenabdeckgitters aus Fig. 1,
- Fig. 5: eine Draufsicht auf das Augenhöhlenabdeckgitter aus Fig. 1,
- Fig. 6: eine Rückansicht auf das Augenhöhlenabdeckgitter aus Fig. 1,
- Fig. 7: das Vorhalten einer bogenförmigen Schnittlinie zum Erleichtern des Anpassens des Augenhöhlenabdeckgitters der Fig. 1 bis 6 durch Entfernung eines medialen Flügels im Rahmen einer Perforationslinie,
- Fig. 8: eine weitere Variante zum Vorhalten von Schnittmöglichkeiten an einem Posteriorabschnitt mit (Neben-)Schnittlinien, die zueinander parallel angeordnet sind und eine Hauptschnittlinie kreuzen,
- Fig. 9: das Vorhalten von Perforationslinien zum teilweisen Entfernen des medialen Flügels,
- Fig. 10: eine mediale Flügelperforationslinie und eine posterior gelegene Verkleinerungslinie, die sichelförmig ausgebildet sind,
- Fig. 11: eine asymmetrische Schnittlinie am orbitabodenseitigen Ende kombiniert mit einer Medialflügelentfernungslinie, und
- Fig. 12: eine Variante einer Schnittlinienausbildung zum teilweisen Abschneiden der Medialflügelwandung und eines Posteriorsegmentabschnittes.

Die Figuren sind schematischer Natur und dienen nur dem Verständnis der Erfindung.

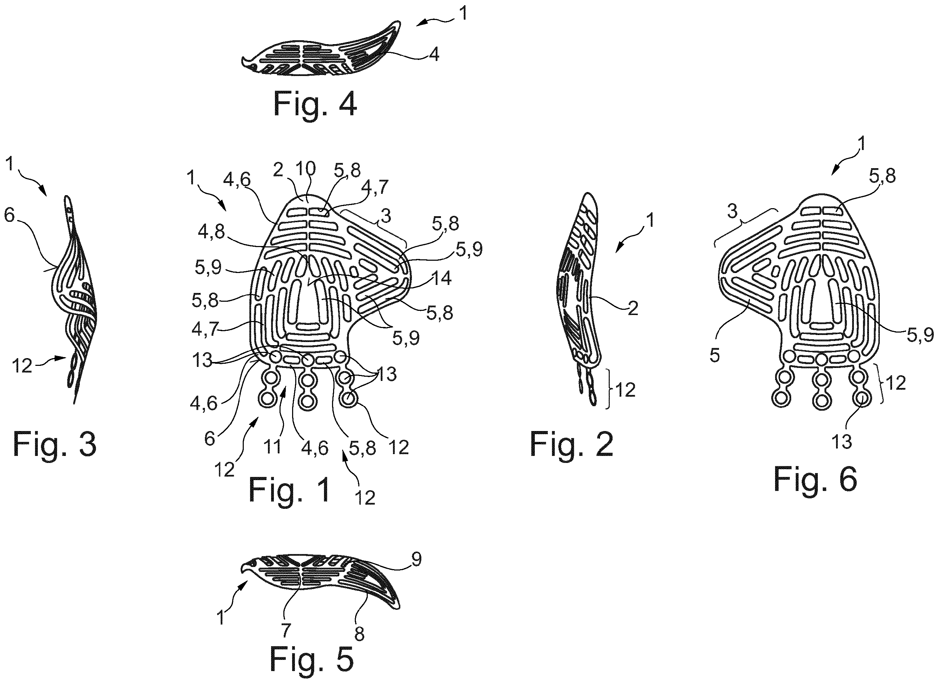

In Fig. 1 ist ein erfindungsgemäßes Augenhöhlenabdeckgitter 1 dargestellt. Das Augenhöhlenabdeckgitter 1 wird geometrisch durch einen Hauptkörper 2 bestimmt, der einen Medialflügel 3 aufweist. Der Hauptkörper 2 bildet Stege 4 aus, die zueinander durch Ausnehmungen/Leerräume 5 voneinander beabstandet sind. Der äußerste Steg 4 formt eine umlaufende Abschlusskante 6 aus, die auch als Einfassung bezeichnet werden kann. Die nicht auf der Abschlusskante endenden Stege werden auch als innere Stege 7 bezeichnet.

Die Vielzahl von Ausnehmungen 5 wird unterschieden in erste Ausnehmungen 8 und zweite Ausnehmungen 9. Die Ausnehmungen 8 und 9 sind ausnahmslos länglich, teilweise auch kurvig ausgebildet. Sie haben eine gleichbleibende Breite und sind an ihren Enden abgerundet. Der Hauptkörper 2 weist eine Spitze 10 auf, dem gegenüberliegend ein abgeflachtes Ende 11 ausgebildet ist. Dort ragen drei Laschen 12 nach außen, hier also radial ab. Sie stehen auf der Abschlusskante 6 senkrecht. Jede Lasche 12 weist zwei kreisrunde Löcher 13 auf. Zwischen zwei dem abgeflachten Ende 11 nächstgelegenen ersten Ausnehmungen 8 ist ebenso ein solches Loch 13 vorgesehen. Auch benachbart zu diesem ist unter Zwischenschaltung von jeweils einer länglichen ersten Ausnehmung 8 je ein weiteres Loch 13 ausgebildet.

Im Inneren des Hauptkörpers 2, von fünf zweiten Ausnehmungen 9 umgeben, ist eine bügeleisensohlenartige Struktur vorhanden, die einen Mittelsteg 14 ausformt. Der Mittelsteg 14 hat also angenähert die Form eines Dreiecks mit an den Ecken vorhandenen Abrundungen, wobei zumindest an den Ecken Stege 4 anschließen.

Im Medialflügel 3 sind die ersten Ausnehmungen 8 und die zweiten Ausnehmungen 9 zueinander im spitzen Winkel aufeinander zulaufend ausgerichtet.

Die Figuren 2 bis 6 ermöglichen einen räumlichen Eindruck der in allen Raumrichtungen gebogenen Ausformung des Augenhöhlenabdeckgitters 1.

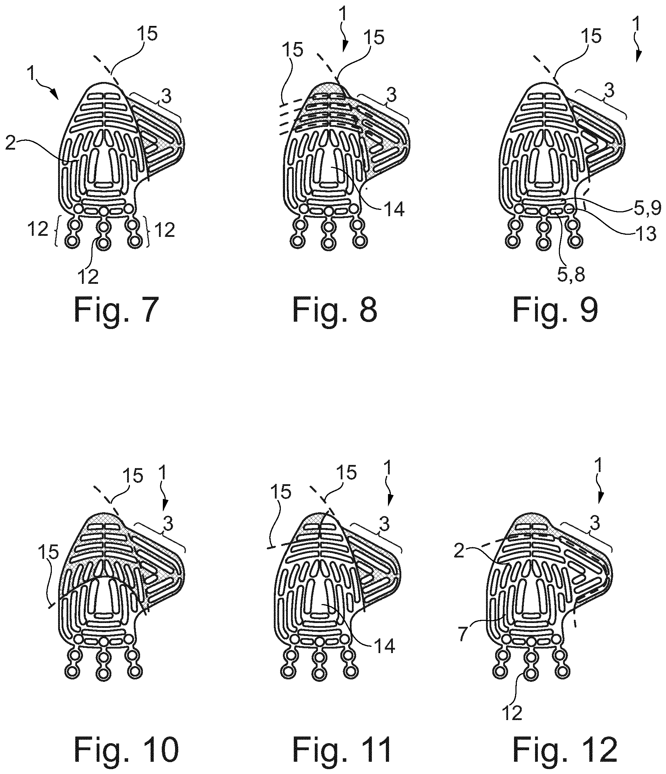

In den Figuren 7 bis 12 ist die stoffliche, geometrische und insbesondere kombinatorische Ausgestaltung der Stege 4 und Ausnehmungen 5 zueinander zum Ausformen von einer imaginären Trennlinie, etwa nach Art einer Sollbruchstellen/Perforationslinien/Sollbruchlinien 15, dargestellt.

In Fig. 8 kreuzen sich einzelne Perforationslinien 15, wohingegen andere zueinander parallel beabstandet bleiben und einer Kurvenform folgen. Der Medialflügel 3 bildet einen Erweiterungsbereich aus.

### Bezugszeichenliste

- 1: Augenhöhlenabdeckgitter
- 2: Hauptkörper
- 3: Medialflügel
- 4: Steg
- 5: Ausnehmung/Leerraum
- 6: Abschlusskante/Einfassung
- 7: innerer Steg
- 8: erste Ausnehmung
- 9: zweite Ausnehmung
- 10: Spitze
- 11: abgeflachtes Ende
- 12: Lasche
- 13: Loch
- 14: Mittel steg
- 15: Imaginäre Trennlinie/Sollbruchstelle/Perforationslinie/Sollbruchlinie

## Patentansprüche

1. Augenhöhlenabdeckgitter (1) mit einem Stege (4) ausbildenden, perforierten und als Platte ausgebildeten, dreidimensional verformbaren und an einen individuellen Schädelknochen anpassbaren Hauptkörper (2), der eine Spitze (10) aufweist und der eine äußere umlaufende Abschlusskante (6) aufweist, wobei mehrere längliche, durch einen Steg (4) von der Abschlusskante (6) getrennte und dort direkt angrenzende erste Ausnehmungen (5, 8) zumindest einem Abschnitt der Abschlusskante (6) folgend angeordnet sind, wobei in Richtung des Zentrums des Gitters (1) mehrere zweite, längliche Ausnehmungen (5, 9) vorhanden sind, die die gleiche Ausrichtung wie die jeweils benachbarte radial außerhalb von ihr befindliche erste Ausnehmung (5, 8) aufweisen, **dadurch gekennzeichnet, dass** die Stege (4) und Ausnehmungen (5) zur Ausbildung einer imaginären Trennlinie ausgerichtet und beschaffen sind, um eine Sollbruchlinie vorzugeben.

2. Augenhöhlenabdeckgitter (1) nach Anspruch 1, **dadurch gekennzeichnet, dass** die in Radialrichtung gesehen innersten, zweiten Ausnehmungen (5, 9) einer Bügeleisensohlenaußenkontur folgend oder angenähert dreieckig angeordnet sind.

3. Augenhöhlenabdeckgitter (1) nach Anspruch 1 oder 2, **dadurch gekennzeichnet, dass** der Hauptkörper (2) einen als Medialflügel (3) ausgebildeten Erweiterungsbereich aufweist, in dem erste Ausnehmungen (5, 8) in einem spitzen Winkel zueinander angeordnet/ausgerichtet sind.

4. Augenhöhlenabdeckgitter (1) nach einem der Ansprüche 1 bis 3, **dadurch gekennzeichnet, dass** wenigstens eine radial vom Hauptkörper (2) abstehende Lasche (12) vorhanden ist, die zur Aufnahme von Befestigungsmitteln mit einem oder mehreren Löchern (13) versehen ist.

5. Augenhöhlenabdeckgitter (1) nach einem der Ansprüche 1 bis 4, **dadurch gekennzeichnet, dass** die Länge der ersten oder zweiten Ausnehmung (5, 8, 9) mehr als 2,5 mal so groß wie deren Breite ist.

6. Augenhöhlenabdeckgitter (1) nach einem der Ansprüche 1 bis 5, **dadurch gekennzeichnet, dass** das Gitter (1) eine mehrfach gekrümmte Raumform einnimmt.

7. Augenhöhlenabdeckgitter (1) nach einem der Ansprüche 2 bis 6, **dadurch gekennzeichnet, dass** sich eine der zweiten Ausnehmungen (5, 9) bis zu einem die Abschlusskante (6) ausformenden Steg (4) erstreckt.

8. Verfahren zum Herstellen eines Augenhöhlenabdeckgitters (1), nach einem der Ansprüche 1 bis 7, **dadurch gekennzeichnet, dass** unter Nutzung von individuellen Patientendaten eine Negativform geschaffen wird, dann ein vorgefertigtes Rohstück mit ersten und zweiten länglichen Ausnehmungen (5, 8, 9) auf die Negativform aufgelegt wird, dann unter Aufbringung von Kraft/Druck und/oder Wärme das Rohstück an die Negativform angepasst wird, und das abgeformte Augenhöhlenabdeckgitter (1) von der Negativform abgenommen wird.

## Claims

1. Eye socket covering grid (1) having a main body (2) which forms webs (4), is perforated and designed as a plate, can be deformed three-dimensionally, is adaptable to an individual skull bone, which has a tip (10), and which has an outer circumferential end edge (6), wherein a plurality of longitudinal first recesses (5, 8), which are separated from the end edge (6) by a web (4) and are directly adjacent to the end edge (6), are arranged following at least a section of the end edge (6), wherein in the direction of the center of the grid (1) a plurality of second, longitudinal recesses (5, 9) are provided, which have the same orientation as the respective adjacent first recess (5, 8) located radially outside of it, **characterized in that** the webs (4) and recesses (5) are aligned and designed to form an imaginary dividing line in order to define a predetermined breaking line.

2. Eye socket covering grid (1) according to claim 1, **characterized in that** the innermost, second recesses (5, 9), as seen in the radial direction, are arranged following an outer contour of an iron soleplate or approximately triangularly.

3. Eye socket covering grid (1) according to claim 1 or 2, **characterized in that** the main body (2) has an extension region in the form of a medial wing (3), in which first recesses (5, 8) are arranged/aligned at an acute angle to one another.

4. Eye socket covering grid (1) according to one of claims 1 to 3, **characterized in that** there is at least one lug (12) projecting radially from the main body (2) and provided with one or more holes (13) for receiving fastening means.

5. Eye socket covering grid (1) according to one of claims 1 to 4, **characterized in that** the length of the first or second recess (5, 8, 9) is more than 2.5 times its width.

6. Eye socket covering grid (1) according to one of claims 1 to 5, **characterized in that** the grid (1) adopts a multiply curved spatial shape.

7. Eye socket covering grid (1) according to one of claims 2 to 6, **characterized in that** one of the second recesses (5, 9) extends up to a web (4) forming the end edge (6).

8. Method for producing an eye socket covering grid (1) according to one of claims 1 to 7, **characterized in that** a negative mold is created using individual patient data, then a prefabricated blank with first and second longitudinal recesses (5, 8, 9) is placed on the negative mold, then the blank is fitted to the negative mold by applying force/pressure and/or heat, and the molded eye socket covering grid (1) is removed from the negative mold.

## Revendications

1. Grille de recouvrement des orbites (1) pourvue d'un corps principal (2) perforé formant des éléments jointifs (4), réalisé sous la forme d'une plaque, déformable de façon tridimensionnelle et pouvant être ajusté à un os crânien individuel, lequel corps présente une pointe (10) et présente une arête de fermeture (6) périphérique extérieure, dans laquelle plusieurs premiers évidements (5, 8) allongés séparés de l'arête de fermeture (6) par un élément jointif (4) et directement adjacents à cet endroit sont agencés à la suite d'au moins une partie de l'arête de fermeture (6), dans laquelle plusieurs deuxièmes évidements (5, 9) allongés sont présents en direction du centre de la grille (1), lesquels présentent la même orientation que le premier évidement (5, 8) respectivement voisin situé radialement à l'extérieur de ceux-ci, **caractérisée en ce que** les éléments jointifs (4) et les évidements (5) sont orientés de manière à former une ligne de séparation imaginaire et sont de nature à prédéfinir une ligne de rupture.

2. Grille de recouvrement des orbites (1) selon la revendication 1, **caractérisée en ce que** les deuxièmes évidements (5, 9) les plus intérieurs vus dans la direction radiale sont agencés de façon approximativement triangulaire ou de manière à suivre un contour extérieur de semelle de fer à repasser.

3. Grille de recouvrement des orbites (1) selon la revendication 1 ou 2, **caractérisée en ce que** le corps principal (2) présente une zone d'élargissement réalisée sous la forme d'une ailette médiane (3) et dans laquelle les premiers évidements (5, 8) sont situés/orientés les uns par rapport aux autres en formant un angle aigu.

4. Grille de recouvrement des orbites (1) selon l'une quelconque des revendications 1 à 3, **caractérisée en ce qu'**au moins une languette (12) faisant saillie radialement du corps principal (2) est présente, qui, pour loger des moyens de fixation, est pourvue d'un ou de plusieurs trous (13).

5. Grille de recouvrement des orbites (1) selon l'une quelconque des revendications 1 à 4, **caractérisée en ce que** la longueur du premier ou deuxième évidement (5, 8, 9) est plus de 2,5 fois plus grande que la largeur de celui-ci.

6. Grille de recouvrement des orbites (1) selon l'une quelconque des revendications 1 à 5, **caractérisée en ce que** la grille (1) adopte une forme spatiale courbée plusieurs fois.

7. Grille de recouvrement des orbites (1) selon l'une quelconque des revendications 2 à 6, **caractérisée en ce qu'**un des deuxièmes évidements (5, 9) s'étend jusqu'à un élément jointif (4) formant l'arête de fermeture (6).

8. Procédé de fabrication d'une grille de recouvrement des orbites (1), selon l'une quelconque des revendications 1 à 7, **caractérisé en ce qu'**un moule négatif est créé au moyen de données individuelles de patient, puis une ébauche préfabriquée présentant des premiers et deuxièmes évidements (5, 8, 9) allongés est posée sur le moule négatif, puis l'ébauche est ajustée au moule négatif par application d'une force/pression et/ou de chaleur, et la grille de recouvrement des orbites (1) moulée est ôtée du moule négatif.
